**Europäisches Patentamt**

**European Patent Office**

**Office européen des brevets**

(19)

(11) Veröffentlichungsnummer : **0 019 826**
**B1**

(12) ## EUROPÄISCHE PATENTSCHRIFT

(45) Veröffentlichungstag der Patentschrift :
**15.12.82**

(21) Anmeldenummer : **80102758.2**

(22) Anmeldetag : **19.05.80**

(51) Int. Cl.³ : **A 61 B   6/06, G 21 K   1/04**

(54) **Schichtgerät zur Herstellung von Transversalschichtbildern.**

(30) Priorität : **29.05.79 DE 2921820**

(43) Veröffentlichungstag der Anmeldung :
**10.12.80 (Patentblatt 80/25)**

(45) Bekanntmachung des Hinweises auf die Patenterteilung : **15.12.82 Patentblatt 82/50**

(84) Benannte Vertragsstaaten :
**FR GB**

(56) Entgegenhaltungen :
**DE A 2 850 675**
**FR A 2 362 614**
**FR A 2 391 698**
**IEEE TRANSACTIONS ON NUCLEAR SCIENCE,
vol. NS. 16, no. 3, part I, Juni 1969 NEW YORK
(US) C.C. FORSTER : « Shutter and collimator
with rotating radiation-cooled elements for the
beam from the Princeton A.V.F. cyclotron »,
Seiten 622-626**

(73) Patentinhaber : **SIEMENS AKTIENGESELLSCHAFT
Berlin und München
Postfach 22 02 61
D-8000 München 22 (DE)**

(72) Erfinder : **Distler, Walter
Sudetenlandstrasse 23a
D-8520 Erlangen (DE)**
Erfinder : **Kintopp, Erich
Schiestlstrasse 16
D-8520 Erlangen (DE)**

EP 0 019 826 B1

## Schichtgerät zur Herstellung von Transversalschichtbildern

Die Erfindung betrifft ein Schichtgerät zur Herstellung von Transversalschichtbildern eines Aufnahmeobjektes mit einer Strahlenmeßanordnung, die eine Strahlenquelle, welche ein das Aufnahmeobjekt durchdringendes Strahlenbündel erzeugt, dessen Querschnittsausdehnung senkrecht zur Schichtebene gleich der Schichtstärke ist, sowie einen Strahlenempfänger enthält, der die Strahlungsintensität hinter dem Objekt ermittelt, sowie mit einer Drehvorrichtung für die Meßanordnung zur Durchstrahlung des Aufnahmeobjektes aus verschiedenen Richtungen und mit einem Meßwertumformer für die Transformation der vom Strahlenempfänger gelieferten Signale in ein Schichtbild, bei dem zwischen der Strahlenquelle und dem Strahlenempfänger zwei Hohlzylinder mit gleichen Durchmessern und gemeinsamer Achse aus strahlenabsorbierendem Material liegen, zwischen denen ein Spalt zum Durchtritt des Röntgenstrahlenbündels freigelassen und in die das Aufnahmeobjekt einschiebbar ist.

Ein Schichtgerät dieser Art ist in der nicht vorveröffentlichten deutschen Patentanmeldung P 28 50 675.4 beschrieben. Die beiden Hohlzylinder bewirken dabei eine Unterdrückung der Streustrahlung sowohl in dem Fall, in dem der Strahlenempfänger als feststehender Detektorring ausgebildet ist als auch in dem Fall, in dem der Strahlenempfänger mit der Strahlenquelle zusammen zur Abtastung des Aufnahmeobjektes gedreht wird.

Der Erfindung liegt die Aufgabe zugrunde, ein Schichtgerät der eingangs genannten Art so auszubilden, daß die Stärke der untersuchten Schicht einstellbar ist.

Diese Aufgabe ist erfindungsgemäß dadurch gelöst, daß die beiden Hohlzylinder zur Einstellung der Spaltbreite in axialer Richtung verstellbar gelagert sind. Die Spaltbreite bestimmt dabei die Stärke der untersuchten Schicht.

Eine zweckmäßige Ausgestaltung der Erfindung, bei der das Röntgenstrahlenbündel durch keine strahlenabsorbierenden Teile gestört wird, besteht darin, daß die Hohlzylinder an einem Tragring verstellbar geführt sind und daß der dem Tragring abgewandte Hohlzylinder mit seinen am Tragring gelagerten Führungsmitteln über einen für Röntgenstrahlen durchlässigen Zwischenring, der den Spalt überdeckt, verbunden ist. Die Röntgenstrahlung kann dabei ungehindert den Zwischenring durchdringen.

Die Erfindung ist nachfolgend anhand eines in der Zeichnung dargestellten Ausführungsbeispiels näher erläutert. Es zeigen :

Figur 1 einen Schnitt durch ein Schichtgerät nach der Erfindung,

Figur 2 eine Seitenansicht des Tragringes für die Hohlzylinder bei dem Schichtgerät nach Figur 1 mit der Schnittlinie I-I für die Figur 1, und

Figur 3 einen Schnitt nach der Linie III-III in Figur 2.

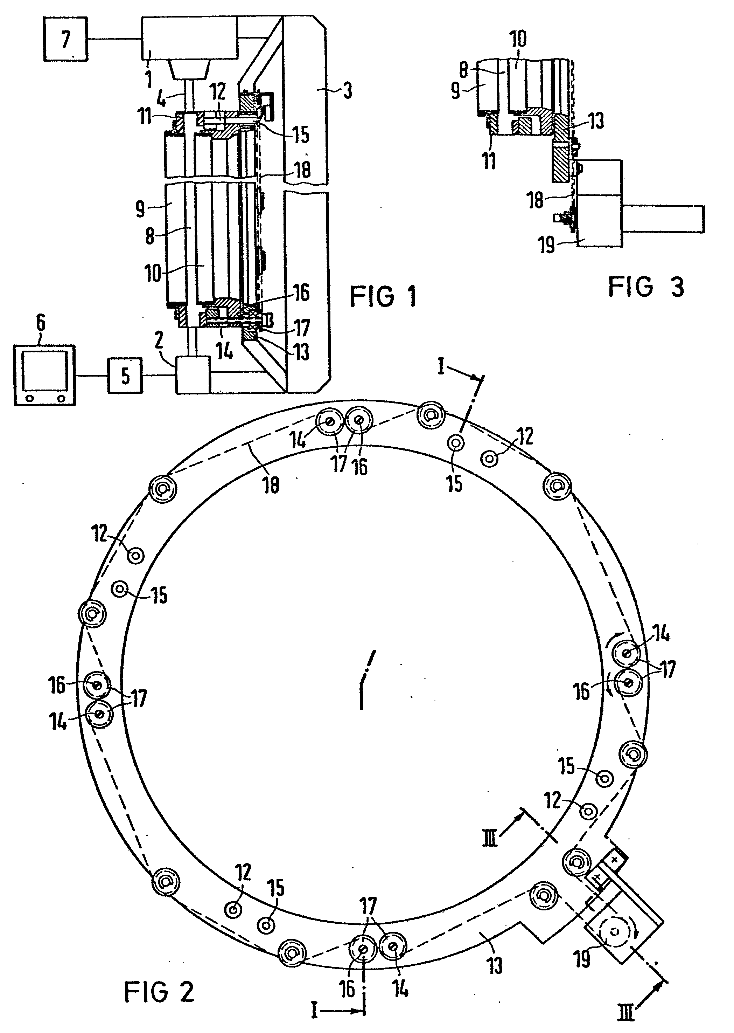

In der Figur 1 ist eine Röntgenröhre 1 und ein Strahlenempfänger 2 dargestellt, welche an einem Drehring 3 befestigt sind. Die Röntgenröhre 1 sendet ein senkrecht zur Zeichenebene fächerförmiges Strahlenbündel 4 aus, das von den Detektoren des Strahlenempfängers 2 empfangen wird. Die Zahl der in einer Reihe liegenden Detektoren des Strahlenempfängers 2 und der Öffnungswinkel des Strahlenbündels 4 sind dabei so aufeinander abgestimmt, daß eine Querschicht eines zwischen der Röntgenröhre 1 und dem Strahlenempfänger 2 angeordneten Aufnahmeobjektes von Röntgenstrahlung durchsetzt wird. Das Aufnahmeobjekt wird abgetastet, indem der Drehring 3 mit der Röntgenröhre 1 und dem Strahlenempfänger 2 um einen Winkel von 360° gedreht wird. Die Ausgangssignale des Strahlenempfängers 2, die dabei erzeugt werden, werden von einem einen Computer enthaltenden Meßwertumformer 5 empfangen, der daraus das Bild der untersuchten Querschicht des Aufnahmeobjektes berechnet und seine Wiedergabe auf einem Sichtgerät 6 bewirkt. Die Röntgenröhre 1 wird von einem Röntgengenerator 7 versorgt.

Die Dicke der untersuchten Querschicht des Aufnahmeobjektes ist durch die Breite eines Spaltes 8 bestimmt, der zwischen zwei Hohlzylindern 9, 10 aus strahlenabsorbierendem Material, z.B. aus Blei, freigelassen ist. Der Hohlzylinder 9 ist über einen Zwischenring und zwar einen Kunststoffring 11, der für Röntgenstrahlung durchlässig ist und den Spalt 8 überdeckt, mit Säulenführungen 12 verbunden, die in einem Tragring 13 geführt sind. Der Tragring 13 ist am Drehring 3 befestigt. Ferner ist der Kunststoffring 11 mit Gewindespindeln 14 verbunden, die ebenfalls im Tragring 13 geführt sind.

Ähnlich wie der Hohlzylinder 9 ist auch der Hohlzylinder 10 am Tragring 13 gelagert. Er ist einerseits durch Säulenführungen 15 und andererseits durch Gewindespindeln 16 am Tragring 13 geführt.

Wie insbesondere aus der Figur 2 hervorgeht, sitzen auf den Gewindespindeln 14 und 16 Ritzel 17, über die ein Kettenzug 18 geführt ist. Der Kettenzug 18 ist noch über am Tragring 13 geführte, in der Figur 2 nicht näher bezeichnete Umlenkrollen geführt und wird von einem in den Figuren 2 und 3 sichtbaren Antriebsmotor 19 angetrieben. Die Gewinde der Gewindespindeln 14 und 16 sind so gewählt, daß beim Antrieb des Kettenzuges 18 in der einen Richtung die Hohlzylinder 9, 10 aufeinander zu und beim Antrieb in der anderen Richtung voneinander bewegt werden. Über den Antrieb 19 ist also die Spaltbreite zwischen den Hohlzylindern 9 und 10 und damit die Schichtdicke einstellbar. Sie unterdrücken in jeder Stellung die von dem in das Innere der Hohlzylinder 9 und 10 eingeschobenen Aufnahmeobjekt ausgehende Streustrahlung. Das Aufnahmeobjekt kann dabei im Bedarfsfall durch den Tragring 13 und den Drehring 3 hindurchgeschoben werden.

Aus der Figur 1 geht hervor, daß die beiden Hohlzylinder 9 und 10 gleiche Durchmesser und eine gemeinsame Achse besitzen. Der dem Tragring 13 abgewandte Hohlzylinder 9 ist mit seinen am Tragring gelagerten Führungsmitteln 12, 14 über den Kunststoffring 11 verbunden.

### Ansprüche

1. Schichtgerät zur Herstellung von Transversalschichtbildern eines Aufnahmeobjektes mit einer Strahlenmeßanordnung, die eine Strahlenquelle, welche ein das Aufnahmeobjekt durchdringendes Strahlenbündel erzeugt, dessen Querschnittsausdehnung senkrecht zur Schichtebene gleich der Schichtstärke ist, sowie einen Strahlenempfänger enthält, der die Strahlungsintensität hinter dem Objekt ermittelt, sowie mit einer Drehvorrichtung für die Meßanordnung zur Durchstrahlung des Aufnahmeobjektes aus verschiedenen Richtungen und mit einem Meßwertumformer für die Transformation der vom Strahlenempfänger gelieferten Signale in ein Schichtbild, bei dem zwischen der Strahlenquelle und dem Strahlenempfänger zwei Hohlzylinder mit gleichen Durchmessern und gemeinsamer Achse aus strahlenabsorbierendem Material liegen, zwischen denen ein Spalt zum Durchtritt des Röntgenstrahlenbündels freigelassen und in die das Aufnahmeobjekt einschiebbar ist, dadurch gekennzeichnet, daß die beiden Hohlzylinder (9, 10) zur Einstellung der Spaltbreite in axialer Richtung verstellbar gelagert sind.

2. Schichtgerät nach Anspruch 1, dadurch gekennzeichnet, daß die Hohlzylinder (9, 10) an einem Tragring (13) verstellbar geführt sind.

3. Schichtgerät nach Anspruch 2, dadurch gekennzeichnet, daß der dem Tragring (13) abgewandte Hohlzylinder (9) mit seinen am Tragring (13) gelagerten Führungsmitteln (12, 14) über einen für Röntgenstrahlen durchlässigen Zwischenring (11), der den Spalt (8) überdeckt, verbunden ist.

### Claims

1. Tomograph apparatus for the production of transversal layer images of an object to be photographed comprising a radiation measuring arrangement containing a beam source producing a focussed beam having a cross-section perpendicular to the layer plane whose expansion equals the layer thickness, which arrangement contains a radiation sensor which determines the radiation intensity behind the object, and further comprising a rotary device for the measuring arrangement enabling the object to be photographed to be irradiated from different directions, and comprising a test value modulator for the transformation of the signals supplied by the radiation sensor into a layer image, wherein between the beam source and the radiation sensor, there are arranged two hollow cylinders of equal diameter on a common axis and made of a material which absorbs radiation, and a gap between the hollow cylinders kept free for the penetration of the X-ray beam to the object to be photographed inserted into these hollow cylinders, characterised in that the two hollow cylinders (9, 10) are arranged so as to be adjustable in order to adjust the width of the gap in the axial direction.

2. Apparatus as claimed in Claim 1, characterised in that the hollow cylinders (9, 10) are adjustably guided on a supporting ring (13).

3. Apparatus as claimed in Claim 3, characterised in that the hollow cylinder (9) which faces away from the supporting ring (13) is connected to its guide means (12, 14), which rest on the supporting ring (13), by means of an intermediate ring (11) which allows the X-rays to penetrate and covers the gap (8).

### Revendications

1. Appareil pour la prise de vues en couches en vue de l'établissement de tomographies d'un objet de prise de vue, à dispositif de mesure du rayonnement comportant une source de rayonnement qui produit un faisceau de rayonnement traversant l'objet de prise de vues et dont l'étendue de sa section transversale, perpendiculairement au plan de la couche, est égale à l'épaisseur de la couche, ainsi qu'un récepteur de rayonnement qui détermine l'intensité du rayonnement à l'arrière de l'objet, et un dispositif de rotation pour le dispositif de mesure en vue de l'irradiation de l'objet de prise de vues à partir de différentes directions, et avec un convertisseur de valeurs de mesures pour transformer les signaux fournis par le récepteur de rayonnement en une tomographie, dans lequel, se situent, entre la source de rayonnement et le récepteur de rayonnement, deux cylindres creux d'un même diamètre, d'axes communs et réalisés avec un matériau absorbant le rayonnement, une fente étant dégagée entre ces cylindres pour le passage du faisceau de rayons X, alors que dans ces derniers est susceptible d'être engagé l'objet de prise de vues, caractérisé par le fait que les deux cylindres creux (9, 10) sont montés de façon réglable en direction axiale, en vue d'ajuster la largeur de la fente.

2. Appareil de prise de vues en couches selon la revendication 1, caractérisé par le fait que les cylindres creux (9, 10) sont guidés sur un anneau de support (13) de manière à pouvoir y être déplacés.

3. Appareil pour la prise de vues en couches selon la revendication 2, caractérisé par le fait que le cylindre creux (9) qui est éloigné de l'anneau de support (13) est relié par ses moyens de guidage (12, 14) montés sur l'anneau de support (13), par l'intermédiaire d'un anneau intermédiaire (11) perméable aux rayons X et recouvrant la fente (8).

79 P 5049

FIG 1

FIG 3

FIG 2